# EUROPÄISCHE PATENTANMELDUNG

(11) **EP 1 541 577 A1**
(43) Veröffentlichungstag der Anmeldung: **15.06.2005**
(21) Anmeldenummer: 04028197.4
(22) Anmeldetag: 27.11.2004
(51) Int. Cl.: C07D 519/00, C08G 61/12

(54) **Multifunktionelle 3,4-Alkylendioxythiophen-Derivate und diese enthaltende elektrisch leitfähige Polymere**

(30) Priorität: 10.12.2003 DE 10357571
(71) Anmelder: H.C. Starck GmbH, 38642 Goslar (DE)
(72) Erfinder: Heuer, Helmut-Werner, Dr., 47829 Krefeld (DE); Wehrmann, Rolf, Dr., 47800 Krefeld (DE)
(74) Vertreter: Bramer-Weger, Elmar, Dr

(57) **Zusammenfassung**

Multifunktionelle 3,4-Alkylendioxythiophen-Derivate der Formel (I) 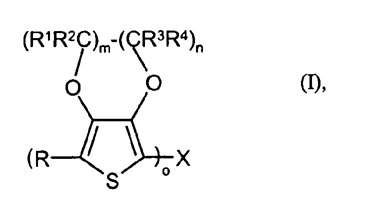 und deren Verwendung zur Herstellung von elektrisch leitfähigen Oligo- oder Polymeren, sowie Oligo- oder Polymere, die diese 3,4-Alkylendioxythiophen-Derivate als Wiederholungs- bzw. Vernetzungseinheit enthalten.

## Beschreibung

Die Erfindung betrifft multifunktionelle 3,4-Alkylendioxythiophen-Derivate, ein Verfahren zu deren Herstellung und deren Verwendung zur Herstellung von elektrisch leitfähigen, insbesondere langkettenverzweigten Oligo- oder Polymeren. Des weiteren betrifft die Erfindung Oligo- oder Polymere, die diese Verbindungen als Wiederholungseinheit bzw. als Vemetzungseinheit enthalten, insbesondere langkettenverzweigte elektrisch leitfähige Polymere.

Organische leitfähige Polymere besitzen ein breites Anwendungsspektrum. Beispielhaft sei der Einsatz zur Herstellung von Polymerbatterien, von Dioden oder Transistoren oder von Solarzellen, aber auch von Kondensatoren, von organischen oder anorganischen Leuchtdioden, ITO- bzw. TCO-Ersatz (ITO = Indium-Zinn-Oxid, TCO = transparent conductive oxide), Displays wie LCDs oder PDLCs oder elektrochromen Vorrichtungen genannt. Weitere Anwendungen sind Korrosionsschutz, Antistatik, Sensortechnik sowie als Lochinjektions- und Glättungsschicht auf TCO-Substraten. Als organische leitfähige Polymere finden beispielsweise Systeme auf Basis von Polyacetylen, Poly(p-phenylen), Polythiophen oder Polypyrrol Anwendung.

Es sind einige elektrisch leitfähige Oligo- bzw. Polymere bekannt, die aus Thiophenderivaten hergestellt werden. Ein besonderes Beispiel ist das Poly[3,4-ethylendioxythiophen] (PEDT), das insbesondere in der kationischen Form mit Polystyrolsulfonsäure (PSS) als weiterer anionischer Komponente eingesetzt wird. PEDT-PSS ist unter der Bezeichnung Baytron® P kommerziell erhältlich.

Um die Polymereigenschaften, insbesondere den Polymerisationsgrad, die Filmbildungseigenschaften und die Löslichkeit und Leitfähigkeit des Polymers gezielt auf die jeweiligen Anforderungen einstellen zu können, ist es notwendig, eine Vielzahl geeigneter Monomer-Bausteine zur Verfügung zu haben. Der Polymerisationsgrad lässt sich besonders effektiv durch den Einbau von Verzweigungs- bzw. Vernetzungsstellen in das Polymer beeinflussen. Es war daher Aufgabe der vorliegenden Erfindung, neue multifunktionelle Thiophen-Derivate zur Verfügung zu stellen, die als Verzweigungs- bzw. Vernetzungsstellen in Polymeren fungieren können. Durch den Einsatz dieser multifunktionellen Thiophenbausteine in Mischung mit den gängigen bifunktionellen Bausteinen lassen sich bei der Copolymerisation kurzkettenverzweigte und langkettenverzweigte elektrisch leitfähige Polymere herstellen.

Unter langkettenverzweigt werden dabei solche elektrisch leitfähigen Polymere verstanden, die auf der Polymerhauptkette Seitenketten besitzen, welche in Bezug auf die Längengrößenordnung der Länge der Polymerhauptkette entsprechen können. Kurzkettenverzweigte Polymere zeigen dagegen nur kürzere aufgepfropfte Seitenketten auf der Polymerhauptkette.

Diese Arten von verzweigten elektrisch leitfähigen Polymeren auf Basis von multifunktionellen 3,4-Alkylendioxythiophen-Derivaten sind bisher nicht beschrieben.

Es konnten nun multifunktionelle 3,4-Alkylendioxythiophen-Derivate hergestellt werden, wobei jeweils drei oder vier 3,4-Alkylendioxythiophen-Reste an eine gemeinsame Verknüpfungseinheit gebunden sind.

Gegenstand der Erfindung sind also Verbindungen der Formel (I) 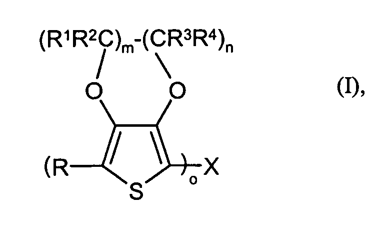 wobei
- n: und m unabhängig voneinander für eine ganze Zahl von 1 bis 5,
- o: für 3 oder 4,
- R', R², R³ und R⁴: jeweils unabhängig voneinander für Wasserstoff, Halogen, gegebenenfalls substituiertes C₁-C₂₀-Alkyl, Hydroxyl, einen gegebenenfalls substituierten Esterrest -(CH₂)ₚ-O-CO-C_{q}H_{2q+1}, einen gegebenenfalls substituierten Etherrest -(CH₂)ₚ-O-C_{q}H_{2q+1}, einen gegebenenfalls substituierten Rest -(CH₂)ₚ-O-C_{q}H_{2q}SO₃Na, einen gegebenenfalls substituierten Oligoethylenglycolrest -(CH₂)ₚ-O-(CO)ᵣ-(CH₂CH₂O)ₛCH₃, einen eine mesogene Gruppe enthaltenden Rest oder zusammen für eine Kronenetherstruktur stehen, wobei
- p: jeweils eine ganze Zahl von 0 bis 6,
- q: jeweils eine ganze Zahl von 1 bis 20,
- r: 0 oder 1 und
- s: eine ganze Zahl von 1 bis 20 bedeuten,
und
- R: für Wasserstoff oder ein Thiophen der Formel (II) 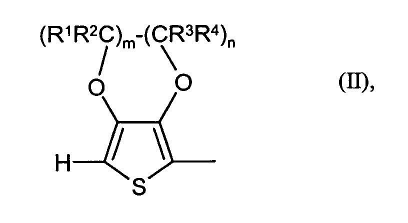
wobei
- m, n, R¹, R², R³ und R⁴: die oben angegebene Bedeutung haben, und
- X: für eine Verknüpfungseinheit stehen.

Unter "substituiert" ist hier und im Folgenden, wenn nicht anders erwähnt, insbesondere halogensubstituiert zu verstehen. Besonders bevorzugt ist eine Substitution mit F, Br, Cl oder insbesondere bevorzugt mit F.

Vorzugsweise bedeuten n und m jeweils 1.

R¹, R², R³ und R⁴ stehen bevorzugt jeweils unabhängig voneinander für Wasserstoff, Halogen, bevorzugt Fluor oder Chlor, C₁-C₁₄-Alkyl, CH₂OH, CH₂O(CO)CₜHal₂ₜ₊₁, CH₂OCₜH₂ₜ₊₁, CH₂OCₜHal₂ₜ₊₁, CH₂O(CH₂CH₂O)ₜCH₃ oder CH₂O(CH₂)ₜSO₃Na, wobei t jeweils für eine ganze Zahl von 1 bis 20 und Hal für Halogen, vorzugsweise für Fluor, steht.

R¹ R², R³ und R⁴ stehen insbesondere bevorzugt für Wasserstoff.

R steht vorzugsweise für Wasserstoff.

Insbesondere bevorzugt handelt es sich bei der Thiophen-Einheit demnach um eine 3,4-Ethylendioxythiophen-Einheit.

Bei der Verknüpfungseinheit X kann es sich um jede Einheit handeln, an die 3 bzw. 4 Thiophen-Einheiten chemisch gebunden werden können. Beispielhaft seien C₅-C₁₄-Aryl, C₁₂-C₂₀-Biaryl, gegebenenfalls substituiertes Triarylamin und gegebenenfalls substituiertes Triarylphosphan genannt.

In einer speziellen Ausführungsform steht o für 3 und es handelt sich bei der Verknüpfungseinheit X um einen Benzolrest oder einen Triphenylaminrest.

In einer weiteren Ausführungsform steht o für 4 und es handelt sich bei der Verknüpfungseinheit X um einen Biphenylrest.

Besonders bevorzugt handelt es sich bei dem erfindungsgemäßen, multifunktionellen 3,4-Alkylendioxythiophen-Derivat um eine Verbindung der Formel (III) 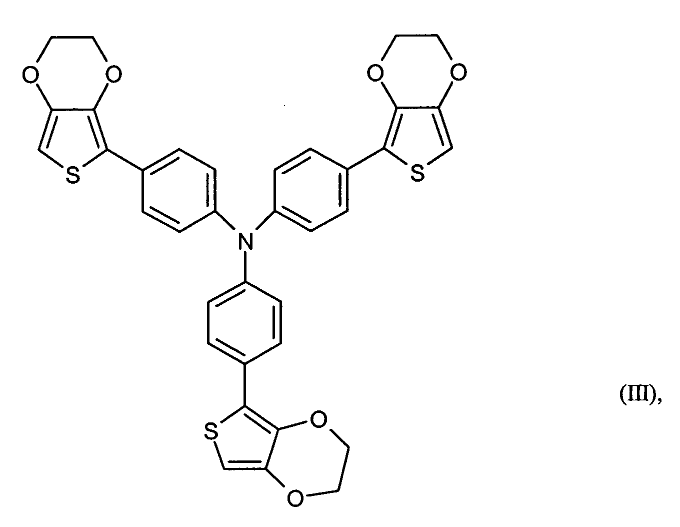 eine Verbindung der Formel (IV) 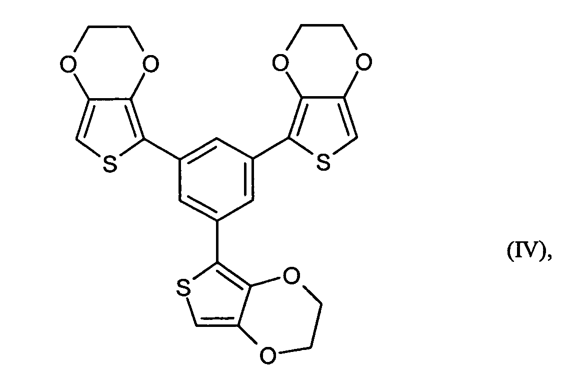 oder eine Verbindung der Formel (V)

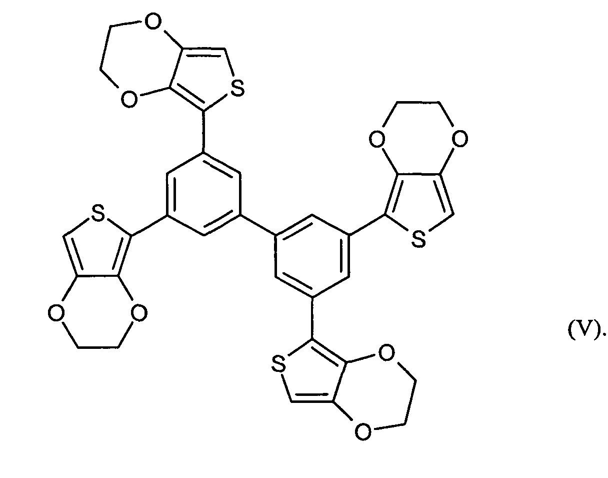

Verbindungen der Formeln (I), (III), (IV) und (V) können beispielsweise durch Reaktion geeigneter Thiophene mit einer halogensubstituierten Verknüpfungseinheit hergestellt werden.

Dabei wird beispielsweise zunächst das gewünschte Thiophenderivat der Formel (VII) 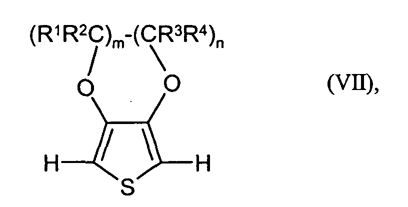 wobei m, n, R¹, R², R³ und R⁴ die oben angegebene Bedeutung haben, durch Umsetzung mit beispielsweise ca. einem Moläquivalent Base, beispielsweise *tert*-Butyllithium in die Monolithiumverbindung und diese anschließend durch Umsetzung mit einem Trialkylzinnhalogenid, vorzugsweise mit Trimethylzinnbromid, Trimethylzinnchlorid, Tri-tert-butylzinnbromid oder Tritert-butylzinnchlorid in eine Verbindung der Formel (VIII) 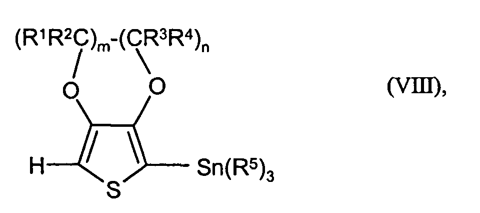 wobei m, n, R¹, R², R³ und R⁴ die oben angegebene Bedeutung haben und R⁵ für C₁-C₁₀-Alkyl steht, überführt.

Vorzugsweise steht R⁵ für Methyl oder *tert*-Butyl.

Die Verbindung der Formel (VIII) kann beispielsweise in Gegenwart eines Pd(0)-Katalysators mit einer halogensubstituierten Verknüpfungseinheit zum gewünschten Produkt der Formel (I) umgesetzt werden.

Diese Umsetzung kann beispielsweise in dipolaren, aprotischen Lösemitteln, wie beispielsweise N-Methyl-2-pyrrolidon (NMP), Dimethylformamid, Dimethylacetamid, Dimethylsulfoxid oder hochsiedenden Ketonen durchgeführt werden. Bevorzugt wird als Lösungsmittel Dimethylformamid eingesetzt.

Die Umsetzung kann beispielsweise bei einer Temperatur von 20 bis 150°C durchgeführt werden. Bevorzugt erfolgt die Reaktion bei Normaldruck. Es kann aber auch unter erhöhtem Druck gearbeitet werden.

Die Verbindungen der Formeln (I), (IM), (IV) und (V) lassen sich zur Herstellung von elektrisch leitfähigen Oligo- und Polymeren verwenden. Dabei kann sowohl nur eine dieser Verbindungen als Monomer eingesetzt werden, als auch ein Gemisch verschiedener Verbindungen, die unter die Definition der Formeln (I), (III), (IV) und/oder (V) fallen.

Werden ausschließlich Verbindungen der Formeln (I), (III), (IV) und/oder (V) als Monomer eingesetzt, so werden hochverzweigte Netzwerkstrukturen erhalten.

Vorzugsweise werden die Verbindungen der Formeln (I), (III), (IV) und/oder (V) jedoch nicht zur Herstellung von elektrisch leitfähigen Oligo- und Polymeren verwendet, die ausschließlich aus solchen Einheiten aufgebaut sind, sondern gezielt als Vernetzungseinheiten bei der Herstellung von elektrisch leitfähigen Oligo- und Polymeren eingebracht, die neben einer oder mehrerer Verbindungen der Formeln (I), (III), (IV) und/oder (V) auch weitere, difunktionelle Thiophen-Derivate als Monomere enthalten, insbesondere 3,4-Ethylendioxythiophen. Der Vemetzungsgrad des Oligooder Polymers hängt insbesondere vom Molverhältnis der erfindungsgemäßen multifunktionellen 3,4-Alkylendioxythiophen-Derivate zu der Menge an eingesetzten difunktionellen Thiophen-Derivaten ab, und kann durch Wahl des Molverhältnisses entsprechend eingestellt werden.

Die Polymerisation erfolgt entsprechend dem Vorgehen bei der Polymerisation bekannter Thiophen-Derivate. Sie kann beispielsweise oxidativ mit Oxidationsmitteln wie Eisen-III-chlorid oder anderen Eisen-III-Salzen, H₂O₂, Natrium- oder Kaliumperoxodisulfat, Kaliumdichromat, Kaliumpermanganat oder elektrochemisch erfolgen.

Die Erfindung betrifft daher weiterhin die Verwendung von Verbindungen der Formeln (I), (III), (IV) und/oder (V) zur Herstellung von elektrisch leitfähigen Oligo- und Polymeren und elektrisch leitfähige Oligo- und Polymere, die durch Polymerisation unter Verwendung einer solchen Verbindung hergestellt werden.

Die Erfindung betrifft insbesondere elektrisch leitfähige Oligo- und Polymere, die als Wiederholungs- bzw. Vemetzungseinheiten Struktureinheiten der Formel (VI) 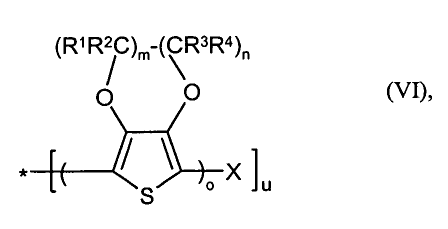 wobei
- n, m, o, R¹, R², R³ und R⁴ und X: die oben angegebenen Bedeutungen haben, und
- u: für eine ganze Zahl von 1 bis 500 steht,
enthalten.

An der mit * gekennzeichneten Position können weitere andere Monomereinheiten verbunden sein, z.B. bei Copolymeren.

Bevorzugt steht u für 1 bis 400, insbesondere bevorzugt für 1 bis 300.

Besonders bevorzugt sind Copolymere mit Struktureinheiten der Formel (VI), die auf Basis von elektrisch leitende Polymere bildenden Monomeren, insbesondere gegebenenfalls substituierten Polythiophenen, besonders bevorzugt 3,4-Ethylendioxithiophen gebildet sind.

Die Struktureinheiten der Formel VI können, wie in der Formel dargestellt, neutral sein. Es ist aber auch möglich, dass sie eine positive Ladung tragen. In diesem Fall erhalten die neuen Polymere Anionen als Gegenionen. Vorzugsweise sind diese Anionen wiederum polymer aufgebaut, insbesondere bevorzugt handelt es sich bei den Polyanionen um Polystyrolsulfonat.

Die erfindungsgemäßen Oligo- und Polymere lassen sich vielseitig einsetzen und finden beispielsweise in verschiedenen Elektronikprodukten Anwendung. Beispielhaft sei der Einsatz zur Herstellung von Polymerbatterien, von Dioden, Photodioden oder Transistoren etwa Feldeffekttransistoren oder von Solarzellen, aber auch Kondensatoren, insbesondere als Elektrodenmaterial in Festelektrolyt-Kondensatoren, organischen der anorganischen Leuchtdioden, die vom Prinzip der Elektrolumineszenz oder der Elektrophosphoreszenz Gebrauch machen, ITO- bzw. TCO-Ersatz (TCO = transparent conductive oxide), Displays wie LCDs oder PDLCs, elektrochromen Vorrichtungen und in der Sensortechnik genannt.

Weitere Anwendungen sind Korrosionsschutz, Antistatik sowie der Einsatz als Lochinjektionsund Glättungsschicht auf TCO-Substraten oder als leitfähige Druckpaste und Injekt-Formulierung. Ferner lassen sich die erfindungsgemäßen Oligo- oder Polymere als Membranen, zur Herstellung wiederaufladbarer Polymerbatterien oder von Schichten auf Papier für Billigelektronikanwendungen oder als Substrate für stromloses Abscheiden verwenden.

### Beispiele:

### Beispiel 1:

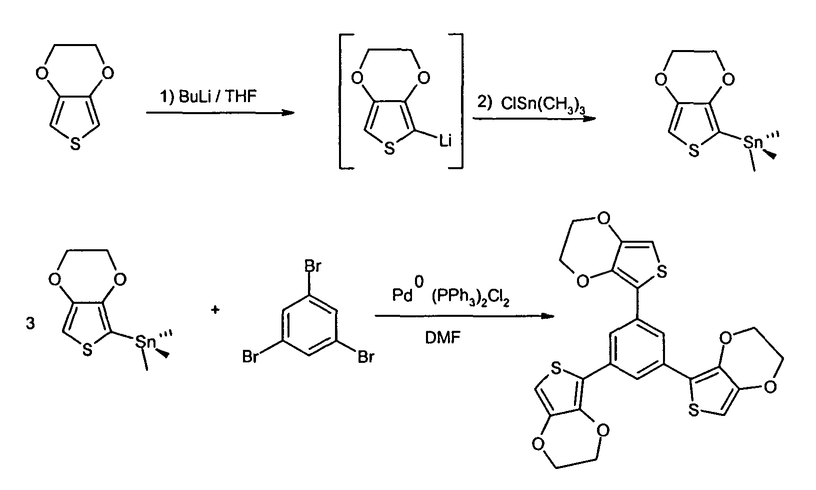

12,78 g (90 mmol) 3,4-Ethylendioxythiophen (Baytron® M, Fa. Bayer AG, Leverkusen) wurden in 100 ml trockenem Tetrahydrofuran (THF) vorgelegt. Nach Kühlung auf -67°C wurden unter Argonschutzgas portionsweise 39 ml einer 15 gew.-%igen Butyllithium (BuLi)-Lösung in n-Hexan zugegeben. Nach beendeter Zugabe wurde noch eine Stunde unter Kühlung nachgerührt. Danach erfolgte die Zugabe von 17,94 g (90 mmol) Trimethylzinnchlorid (Fa. Aldrich) in 90 ml trockenem THF. Man rührte 30 Minuten unter Kühlung nach und lies den Reaktionsansatz dann auf Raumtemperatur erwärmen. Zur Aufarbeitung wurden 200 ml Methylenchlorid hinzugegeben. Die letzten Reste an metallorganischen Verbindungen wurden durch Zugabe von 100 ml Wasser gequencht. Die organische Phase wurde anschließend mit Wasser gewaschen und über Natriumsulfat getrocknet. Nach Abtrennen des Lösungsmittels am Vakuum erhielt man 21,5 g an Rohprodukt, welches im Kühlschrank zur Auskristallisation des Produktes gelagert wurde. Man erhielt 11,5 g eines Feststoffes (Ausbeute: 41,9 % der Theorie) als Produkt.

Analyse mittels Gaschromatographie-Massenspektrometrie (GC-MS) zeigte ein Signal bei einer Molekülmasse von 305, entsprechend dem Molekulargewicht des gewünschten silylierten 3,4-Ethylendioxythiophens. Als Nebenprodukt wurde das zweifach silylierte Produkt erhalten.

Die analoge Reaktion wurde mit folgenden Einwaagen wiederholt:
- 20,19 g (141,9 mmol) 3,4-Ethylendioxythiophen in 150 ml THF
- 60 ml 15 gew.-%iges BuLi in n-Hexan
- 28,35 g (141,9 mmol) Trimethylzinnchlorid

Man erhielt 42,0 g an Rohprodukt.

10,0 g (32,8 mmol) des silylierten 3,4-Ethylendioxythophens wurden in 100 ml trockenem N,N-Dimethylformamid (DMF) unter Argonschutzgas gelöst und mit 3,44 g (10,9 mmol) 1,3,5-Tribrombenzol (Fa. Alrich) und 1,0 g (1,4 mmol) Bis-(triphenylphosphin)-palladium-II-chlorid als Katalysator der Stille-Reaktion versetzt. Nach mehrmaligem Entgasen der gelben Suspension erhitzte man auf 100°C und hielt die Temperatur 15 Stunden lang aufrecht. Zur Aufarbeitung wurde das Lösungsmittel DMF unter Vakuum abgetrennt und der Rückstand über Silicagel chromatographiert.

Man erhielt 0,6 g (Ausbeute: 11 % der Theorie) gelben Feststoff als Produkt.

Außerdem erhielt man als weitere Fraktion 0,1 g eines grünen Feststoffes, welcher als Nebenprodukt folgende Struktur mit einem Molekulargewicht von 714,8 (GC-MS) aufwies:

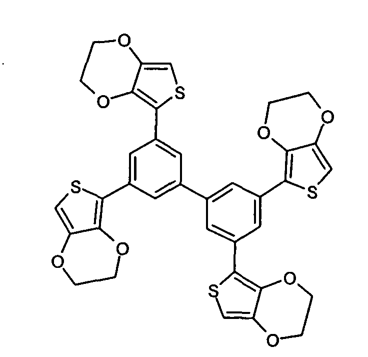

Folgende weitere Strukturen konnten in Spuren nachgewiesen werden:

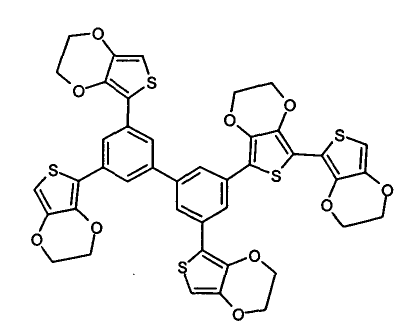

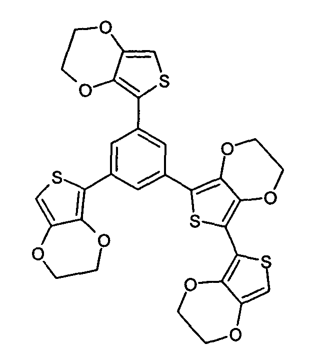

### Beispiel 2:

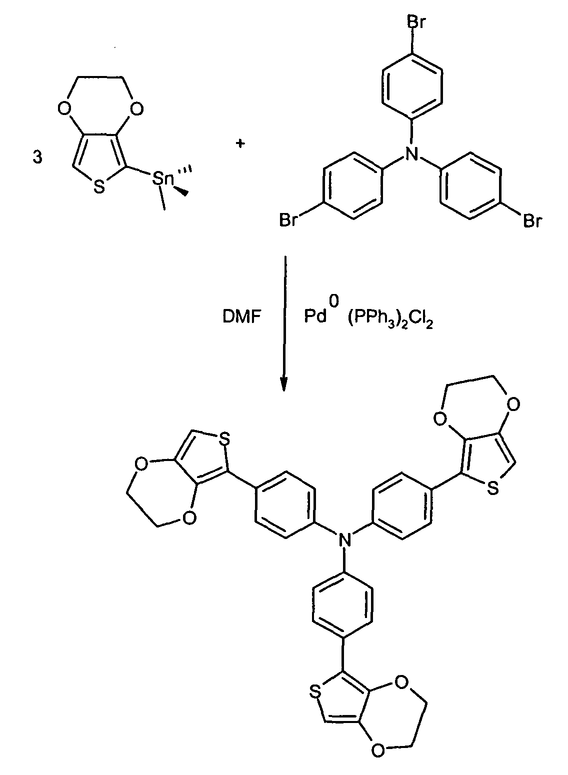

Analog der Kupplungsreaktion aus Beispiel 1 wurden 10,0 g (32,8 mmol) des silylierten 3,4-5 Ethylendioxythiophens aus Beispiel 1 in 100 ml trockenem DMF mit 5,25 g (10,9 mmol) Tris-(4-bromphenyl)amin (Fa. Aldrich) und 1,0 g (1,4 mmol) Bis-(triphenylphosphin)-palladium-II-chlorid umgesetzt. Die Reaktionsmischung wurde für 22,5 Stunden auf 100°C erwärmt. Nach Abtrennen des Lösungsmittels wurde über Silicagel chromatographiert. Man erhielt 0,6 g eines grünen Feststoffes, bei dem es sich um das gewünschte Produkt mit der erwarteten Molekülmasse von 665 handelte (Massenspektrometrie).

### Beispiel 3:

0,025 g (0,05 mmol) des Produktes aus Beispiel 1 wurden in 5 ml trockenem, entgastem Acetonitril mit 0,171 g (0,5 mmol) Tetrabutylammoniumperchlorat als Leitsalz bei 3,5 Volt auf Indiumzinnoxid (ITO) auf Glas potentiostatisch oxidativ elektropolymerisiert. Dabei schied sich ein lilarotbrauner polymerer Film auf dem ITO ab. Nach dem Trocken zeigte das polymere, verzweigte Netzwerk eine dunkelbraun-rote Farbe.

### Beispiel 4:

0,0357 g (0,05 mmol) des tetrafunktionellen Nebenproduktes mit der Masse 714,8 aus Beispiel 1 wurden auf analoge Weise wie unter Beispiel 3 angegeben elektropolymerisiert. Auch hier erhielt man ein polymeres Netzwerk mit dunkelbraun-roter Farbe nach dem Trocknen.

### Beispiel 5:

0,015 g (0,03 mmol) des Produktes aus Beispiel 1 (3 mol-%) und 0,142 g (1 mmol) 3,4-Ethylendioxythiophen (97 mol-%) wurden auf analoge Weise wie unter Beispiel 3 beschrieben, in 100 ml Acetonitril mit 4,4 g (10 mmol) Tetrabutylammoniumperchlorat elektropolymerisiert. Man erhielt ein metallisch glänzendes grau-braunes Polymer.

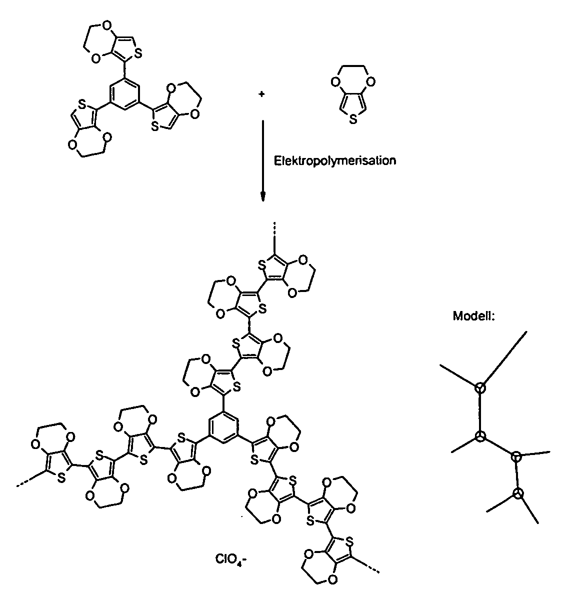

### Beispiel 6:

0,0214 g (0,03 mmol) des Nebenproduktes aus Beispiel 1 (3 mol-%) und 0,142 g (1 mmol) 3,4-Ethylendioxythiophen (97 mol-%) wurden auf analoge Weise wie unter Beispiel 3 beschrieben in 100 ml Acetonitril mit 4,4 g (10 mmol) Tetrabutylammoniumperchlorat elektropolymerisiert. Man erhielt ein metallisch glänzendes dunkelgrau-violettes Polymer.

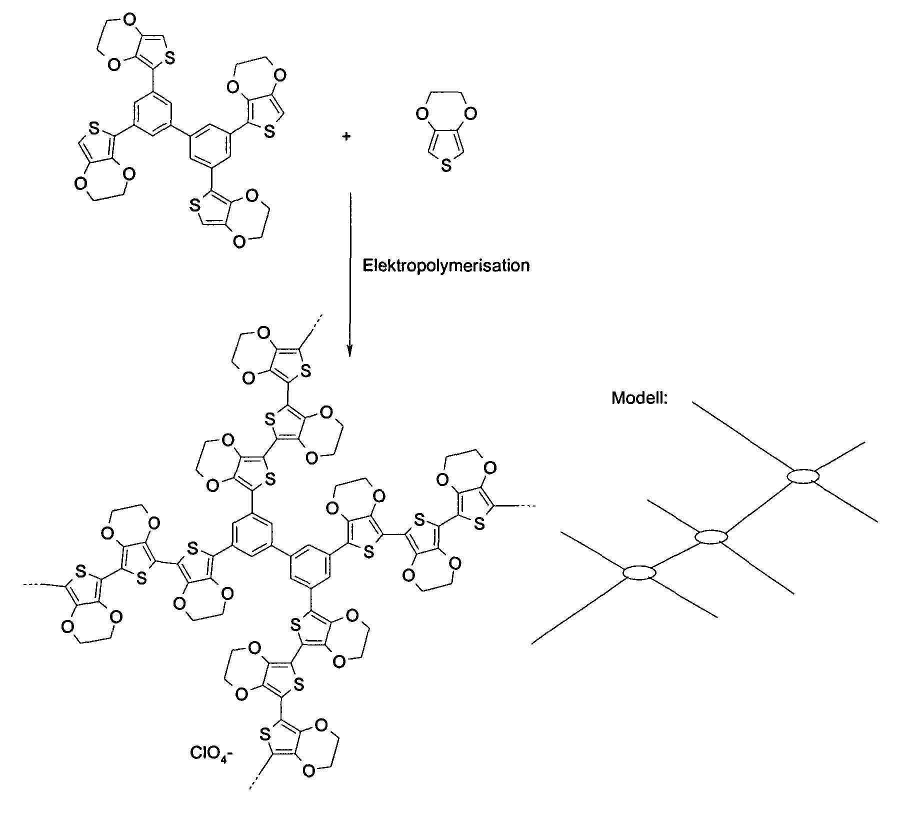

## Patentansprüche

1. Verbindung der Formel (I) 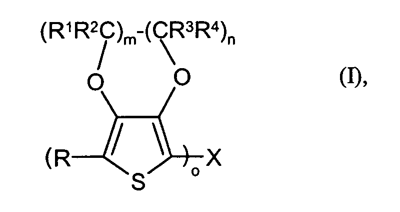 wobei
n und m unabhängig voneinander für eine ganze Zahl von 1 bis 5,
o für 3 oder 4,
R¹, R², R³ und R⁴ jeweils unabhängig voneinander für Wasserstoff, Halogen, gegebenenfalls substituiertes C₁-C₂₀-Alkyl, Hydroxyl, einen gegebenenfalls substituierten Esterrest -(CH₂)ₚ-O-CO-C_{q}H_{2q+1}, einen gegebenenfalls substituierten Etherrest -(CH₂)ₚ-O-C_{q}H_{2q+1}, einen gegebenenfalls substituierten Rest -(CH₂)ₚ-O-C_{q}H_{2q}SO₃Na, einen gegebenenfalls substituierten Oligoethylenglycolrest -(CH₂)ₚ-O-(CO)ᵣ-(CH₂CH₂O)ₛCH₃, einen eine mesogene Gruppe enthaltenden Rest oder zusammen für eine Kronenetherstruktur stehen, wobei
p jeweils eine ganze Zahl von 0 bis 6,
q jeweils eine ganze Zahl von 1 bis 20,
r 0 oder 1 und
s eine ganze Zahl von 1 bis 20 bedeuten,
und
R für Wasserstoff oder ein Thiophen der Formel (II) 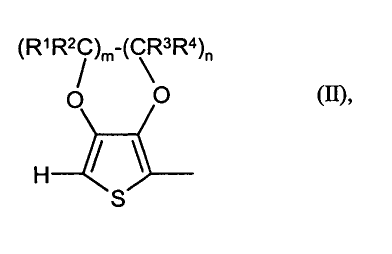
wobei
m, n, R¹, R², R³ und R⁴ die oben angegebene Bedeutung haben, und
X für eine Verknüpfungseinheit, insbesondere eine Verknüpfungseinheit ausgewählt aus der Reihe C₅-C₁₄-Aryl, C₁₂-C₂₀-Biaryl, gegebenenfalls substituiertes Triarylamin und gegebenenfalls substituiertes Triarylphosphan
stehen.

2. Verbindung gemäß Anspruch 1, **dadurch gekennzeichnet, dass** n und m jeweils 1 bedeuten.

3. Verbindung gemäß einem der Ansprüche 1 oder 2, **dadurch gekennzeichnet, dass** R¹, R², R³ und R⁴ jeweils für Wasserstoff stehen.

4. Verbindung gemäß einem der Ansprüche 1 bis 3, **dadurch gekennzeichnet, dass** R für Wasserstoff steht.

5. Verbindung gemäß einem der Ansprüche 1 bis 4, **dadurch gekennzeichnet, dass** o für 3 steht und es sich bei der Verknüpfungseinheit X um einen Benzolrest oder einen Triphenylaminrest handelt.

6. Verbindung gemäß einem der Ansprüche 1 bis 4, **dadurch gekennzeichnet, dass** o für 4 steht und es sich bei der Verknüpfungseinheit X um einen Biphenylrest handelt.

7. Verbindung der Formel (III)

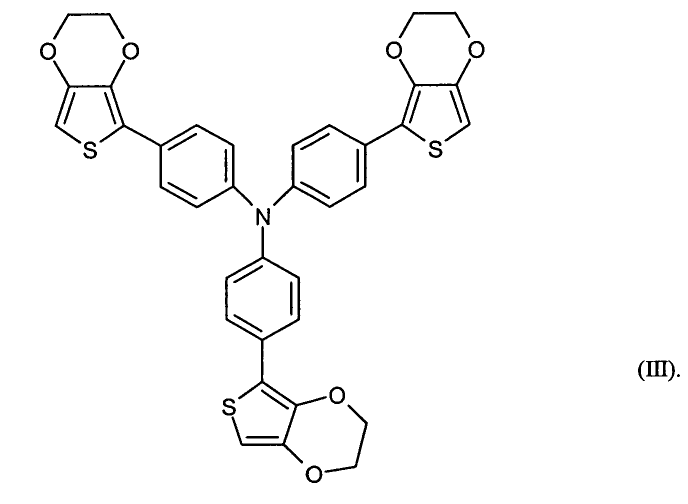

8. Verbindung der Formel (IV)

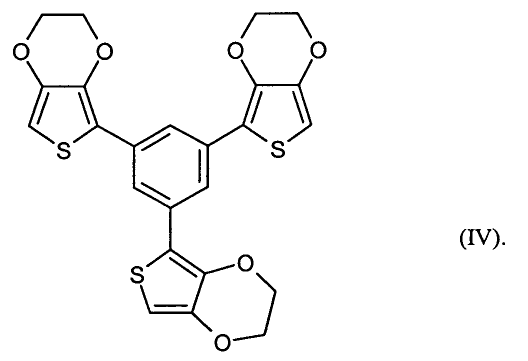

9. Verbindung der Formel (V)

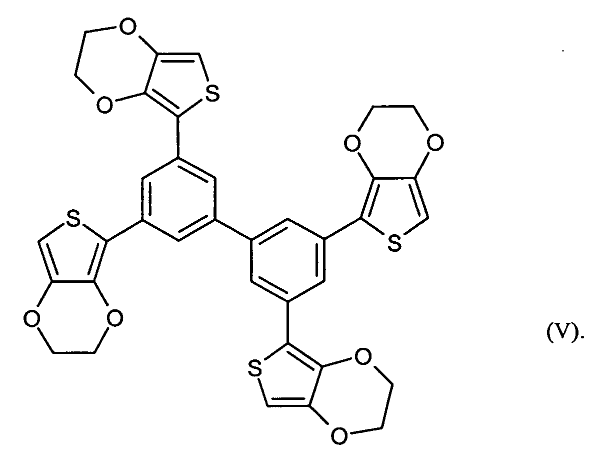

10. Verwendung einer Verbindung gemäß wenigstens eines der Ansprüche 1 bis 9 zur Herstellung eines elektrisch leitfähigen Oligo- oder Polymers.

11. Oligo- oder Polymer enthaltend Struktureinheiten der Formel (VI) 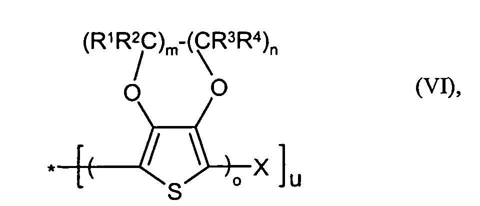 wobei
n und m unabhängig voneinander für eine ganze Zahl von 1 bis 5,
o für 3 oder 4,
u für eine ganze Zahl von 1 bis 500,
R¹, R², R³ und R⁴ jeweils unabhängig voneinander für Wasserstoff, Halogen, gegebenenfalls substituiertes C₁-C₂₀-Alkyl, Hydroxyl, einen gegebenenfalls substituierten Esterrest -(CH₂)ₚ-O-CO-C_{q}H_{2q+1}, einen gegebenenfalls substituierten Etherrest -(CH₂)ₚ-O-C_{q}H_{2q+1}, einen gegebenenfalls substituierten Rest -(CH₂)ₚ-O-C_{q}H_{2q}SO₃Na, einen gegebenenfalls substituierten Oligoethylenglycolrest -(CH₂)ₚ-O-(CO)ᵣ-(CH₂CH₂O)ₛCH₃, einen eine mesogene Gruppe enthaltenden Rest oder zusammen für eine Kronenetherstruktur stehen, wobei
p jeweils eine ganze Zahl von 0 bis 6,
q jeweils eine ganze Zahl von 1 bis 20,
r 0 oder 1 und
s eine ganze Zahl von 1 bis 20 bedeuten,
und
X für eine Verknüpfungseinheit, insbesondere eine Verknüpfungseinheit ausgewählt aus der Reihe C₅-C₁₄-Aryl, C₁₂-C₂₀-Biaryl, gegebenenfalls substituiertes Triarylamin und gegebenenfalls substituiertes Triarylphosphan
steht.

12. Polymer gemäß Anspruch 11, **dadurch gekennzeichnet, dass** das Polymer ein Copolymer auf Basis eines Monomeren ist, welches elektrisch leitfähige Polymere bildet, insbesondere auf Basis eines gegebenenfalls substituierten Thiophens, besonders bevorzugt auf Basis von 3,4-Ethylendioxithiophen, und Struktureinheiten der Formel (VI) aufweist, in der n, m, o, u, R¹, R², R³, R⁴ und X die unter Anspruch 11 angegebene Bedeutung haben.

13. Verwendung eines Oligo- oder Polymers gemäß Anspruch 11 oder 12 zur Herstellung von Produkten für die Elektronikindustrie, insbesondere zur Herstellung von Polymerbatterien, von Dioden oder Transistoren oder von Solarzellen, aber auch Kondensatoren, organischen oder anorganischen Leichtdioden, ITO- bzw. TCO-Ersatz (TCO = transparent conductive oxide), Displays wie LCDs oder PDLCs, elektrochromen Vorrichtungen, Korrosionsschutz, Antistatik, leitfähige Druckpasten, Injekt-Formulierungen sowie als Lochinjektions- und Glättungsschicht auf TCO-Substraten und in der Sensortechnik.
